# EUROPÄISCHE PATENTSCHRIFT

(11) **EP 0 454 816 B1**
(45) Veröffentlichungstag und Bekanntmachung des Hinweises auf die Patenterteilung: **08.06.1994**
(21) Anmeldenummer: 90916703.3
(22) Anmeldetag: 17.11.1990
(51) Int. Cl.: A61M 5/19, A61M 5/32

(54) **INJEKTIONSVORRICHTUNG**
INJECTION DEVICE
DISPOSITIF D'INJECTION

(30) Priorität: 21.11.1989 DE 8913761 U; 03.01.1990 DE 9000024 U; 23.03.1990 DE 9003433 U; 09.08.1990 DE 4025503
(43) Veröffentlichungstag der Anmeldung: 06.11.1991
(73) Patentinhaber: GIP Medizin Technik GmbH, 83224 Grassau (DE)
(72) Erfinder: LINDNER, Andreas, D-8000 München 22 (DE); WECHLER, Ingolf, Max, D-8221 Grabenstätt (DE)
(74) Vertreter: Goddar, Heinz J., Dr.
(86) Internationale Anmeldenummer: DE9000892
(87) Internationale Veröffentlichungsnummer: WO9107197

(56) Entgegenhaltungen:
- EP-A- 0 037 393
- EP-A- 0 156 098
- AT-B- 382 783
- SE-C- 135 247
- US-A- 3 223 083
- US-A- 4 874 368

## Beschreibung

Die Erfindung betrifft eine Injektionsvorrichtung nach dem Oberbegriff des patentanspruches 1.

Solche Injektionsvorrichtungen werden insbesondere zum Injizieren von Zweikomponenten-Fibrinkleber verwendet, wobei Fibrin oder Fibrinogen mit Thrombin innerhalb des menschlichen Körpers an solchen Stellen zur Reaktion gebracht werden sollen, an denen durch Verschließen von Blutgefäßen eine Blutung zum Stillstand gebracht werden muß.

Im Markt sind derartige Fibrinkleber-Injektionsvorrichtungen bekannt, die sich in zwei Typen einordnen lassen. Bei dem einen Typ, wie er beispielsweise aus der dem Oberbegriff des Anspruchs 1 zugrunde liegenden AT-A 382 783 bekannt ist, ist die Injektionshohlnadel an einen Schlauch angeschlossen, der in den Griffstücken einer Kammer mündet, in die ihrerseits die Kanäle der beiden Spritzen-Kupplungsanschlüsse einmünden. Durch Betätigen der beiden Spritzen werden die beiden Fibrinkleberkomponenten gleichzeitig in die Kammer und aus dieser in den Schlauch eingeführt, in welchem sie sich bis zum Erreichen der Injektionshohlnadel vermischen sollen. Einerseits aber ist hierbei keine gründliche Durchmischung der beiden Komponenten gewährleistet, andererseits besteht auch die Gefahr, daß das Komponentengemisch bis zum Erreichen der Injektionshohlnadel aushärtet und dadurch den Schlauch verstopft. Bei dem anderen Vorrichtungstyp werden deshalb zwei Injektionshohlnadeln verwendet, die Seite an Seite miteinander verschweißt sind und jeweils über einen Schlauch mit den Spritzen-Kupplungsanschlüssen verbunden sind, wobei die beiden Schläuche in dem Katheter nebeneinander verlaufen. Hierbei ergibt sich jedoch eine verhältnismäßig große Dicke der Verbundnadeln in Richtung ihrer einander fortsetzenden Diagonalen, wobei die Einzelhohlnadeln mit der ihrer Spitze enthaltenen Seite aneinander angeordnet sind, so daß der Öffnungswinkel der Hohlnadelanordnung verhältnismäßig groß ist, was zu einer unerwünscht ausgeprägten Beschädigung des Gewebes führt. Außerdem ist eine gute Vermischung der beiden Kleberkomponenten nicht gewährleistet, weil die Komponenten aus den injektionsnadeln in das zu behandelnde Gewebe seitlich nebeneinander austreten und die Achsen ihrer Austrittsströme einen verhältnismäßig großen Abstand voneinander haben.

Es ist daher die Aufgabe der Erfindung, eine Injektionsvorrichtung nach dem Oberbegriff dahingehend weiterzuentwickeln, daß ein gutes Durchmischen der Komponenten ohne Gefahr vorzeitigen Aushärtens erzielt wird.

Diese Aufgabe wird bei einer Injektionsvorrichtung der gattungsgemäßen Art durch den im Kennzeichen des Patentanspruches 1 genannten Merkmale gelöst. Vorteilhafte Weiterbildungen der Erfindung sind Gegenstand der Unteransprüche.

Erfindungsgemäß wird diese Aufgabe gelöst durch die im Kennzeichnenden Teil des Anspruchs 1 definierten alternativen Lösungen.

Weiter bevorzugt ist, daß die Injektionsnadel an einer in dem ersten Schlauch befestigten Hülse festgelegt ist, in welcher der zweite Schlauch endet.

Weiterhin kann die der Zahl der Komponenten entsprechende, durch den Katheter verlaufende Anzahl von Schläuchen über jeweils einen Spritzen-Kupplungsanschluß mit einem Griffstück verbunden sein, wobei das Griffstück in den Katheter verschiebbar ausgebildet ist.

Erfindungsgemäß ist für die Injektionsvorrichtung eine einzige Schliffebene der Injektionshohlnadel vorgesehen, unabhängig davon, in welcher Weise die Zuführung der Komponenten geschieht. Es hat sich gezeigt, daß bei einem Winkel der Schliffebene zur Achse der Injektionshohlnadel von etwa 20° der Gewebewiderstand so gering bleibt, daß eine übermäßige Verletzung des Gewebes weitgehend ausgeschlossen ist. Ein solcher optimaler, einheitlicher Winkel wird bei der erfindungsgemäßen Injektionsvorrichtung bei jeder Ausführungsform beibehalten. Gleichzeitig wird festgelegt, daß die zugeführten Komponenten in einer gemeinsamen Ebene münden, wobei in dieser Ebene die Reaktion der Komponenten eingeleitet wird. Die gemeinsame Ebene kann in einem der Schläuche vor der Injektionsnadel liegen, sie kann aber auch mit der Schliffebene der Injektionsnadel übereinstimmen.

Im folgenden soll die Erfindung lediglich beispielhaft anhand der Zeichnungen erläutert werden. Es zeigt:
- Fig. 1: eine Seitenansicht der erfindungsgemäßen Injektionsvorrichtung,
- Fig. 2: einen Teillängsschnitt einer ersten Ausführungsform der Erfindung im Bereich der Injektionshohlnadel in vergrößertem Maßstab,
- Fig. 3: einen Teillängsschnitt einer zweiten Ausführungsform der Erfindung im Bereich der Injektionshohlnadel, und
- Fig. 4: einen Teillängsschnitt einer Ausführungsform der vorliegenden Erfindung im Bereich der Injektionshohlnadel, bei der die Einzelhohlnadeln koaxial angeordnet sind.

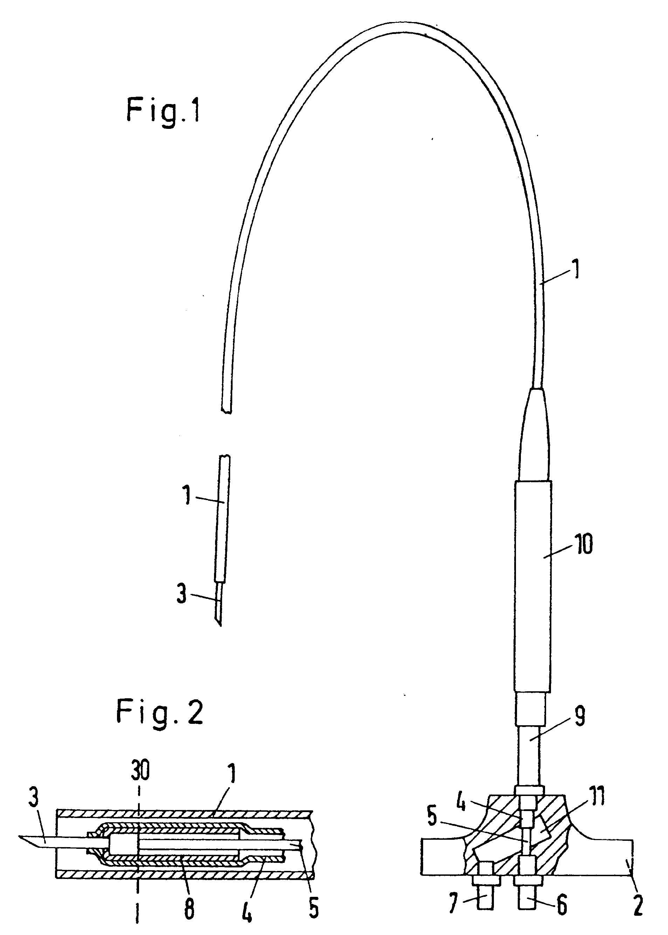

Die in Fig. 1 dargestellte Injektionsvorrichtung weist ein T-förmiges Griffstück 2 auf, welches über eine an ihm befestigte Schieberhülse 9 in einer Griffhülse 10 verschiebbar ist, an deren dem Griffstück 2 abgewandten Ende ein biegsamer schlauchförmiger Katheter 1 festgelegt ist. Im distalen Ende des Katheters 1 ist eine Injektionshohlnadel 3 durch Verschieben der Schieberhülse 9 in der Griffhülse 10 verschiebbar. Durch Hineinschieben der Schieberhülse 9 in die Griffhülse 10 kann somit die Injektionshohlnadel 3 aus dem distalen Ende des Katheters 1 herausgeschoben werden.

Hierzu ist nach Fig. 2 die Injektionsnadel 3 durch eine Hülse 8 verlängert, auf welcher das distale Ende eines ersten Schlauchs 4 festgelegt ist, dessen proximales Ende im Griffstück 2 verankert ist. In der Hülse 8 mündet das distale Ende eines zweiten inneren Schlauches 5 frei aus, dessen Außendurchmesser kleiner ist als der Innendurchmesser des äußeren Schlauches 4 und welcher im letzteren koaxial bis zum Griffstück 2 verläuft. Hier ist der innere Schlauch 5 an einem ersten Spritzen-Kupplungsanschluß 6 angeschlossen, wohingegen der äußere Schlauch in eine Querkammer 11 mündet, durch welche der erste Schlauch 4 hindurchverläuft und in welcher ein zweiter Spritzen-Kupplungsanschluß 7 mündet.

Die Spritzen (nicht gezeigt) sind mit den Kleberkomponenten angefüllt, die somit nach dem Kuppeln der Spritzen mit den Spritzen-Kupplungsanschlüssen 6 und 7 beim gleichzeitigen Betätigen der Spritzkolben in den jeweils zugeordneten Schlauch 4, 5 gepumpt werden und dicht im Abstand vor der Injektionshohlnadel 3 in der Hülse in einer gemeinsamen Ebene 31 zusammentreffen, wo die Reaktion der Komponenten eingeleitet wird, welche im weiteren bis zum Austreten aus der Injektionshohlnadel 3 miteinander vermischt werden. Das Einleiten der Reaktion und das Vermischen erfolgt dicht vor der Injektionshohlnadel und in dieser selbst, wodurch ein vorzeitiges Aushärten der Komponenten vermieden und gleichwohl ein Vermischen vor dem Austreten aus der Injektionshohlnadel in das zu behandelnde Gewebe erreicht wird. Die einläufige Injektionshohlnadel kann hinreichend dünn sein, wodurch keine Komplikation beim Injizieren des Komponentengemisches auftreten können.

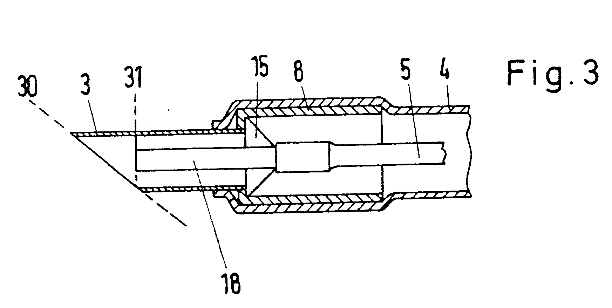

Auch gemäß der Ausführungsform nach Fig. 3 ist die Inkjektionshohlnadel 3 durch eine Hülse 8 verlängert, auf welcher das distale Ende eines ersten Schlauches 4 festgelegt ist, dessen proximales Ende im Griffstück 2 verankert ist. In die Injektionshohlnadel 3 ist als Verlängerung ein Tubus 18 eingesetzt, in den das distale Ende des inneren Schlauches 5 mündet, dessen Außendurchmesser kleiner ist als der Innendurchmesser des äußeren Schlauches 4 und welcher im letzteren praktisch koaxial bis zum Griffstück 2 verläuft. Wiederum ist der innere Schlauch 5 an einen ersten Spritzen-Kupplungsanschluß 6 angeschlossen, wohingegen der äußere Schlauch in eine Querkammer 11 mündet, wie im Zusammenhang mit Fig. 1 ausgeführt. Der Tubus 18 ist konzentrisch zur Injektionsnadel 3 angeordnet und hierzu in der Hülse 8 mittels Stützvorrichtungen 15 festgelegt. Der Tubus 18 endet in geringem Abstand vor der Mündung der Injektionsnadel, wobei die Reaktion der durch die Schläuche 4, 5 zugeführten Komponenten in einer Ebene 31 eingeleitet wird.

Welche der beschriebenen Ausführungsformen gewählt wird, hängt auch davon ab, welche relative Viskosität die zu vermischenden Komponenten miteinander haben. Bei der Anwendung als Fibrinkleber-Injektionsvorrichtung wird man beachten, daß das Fibrinogen dickflüssiger ist und damit eine höhere Viskosität hat als das Thrombin, so daß ein möglichst großer einheitlicher Strömungsquerschnitt für das Fibrinogen zur Verfügung stehen muß.

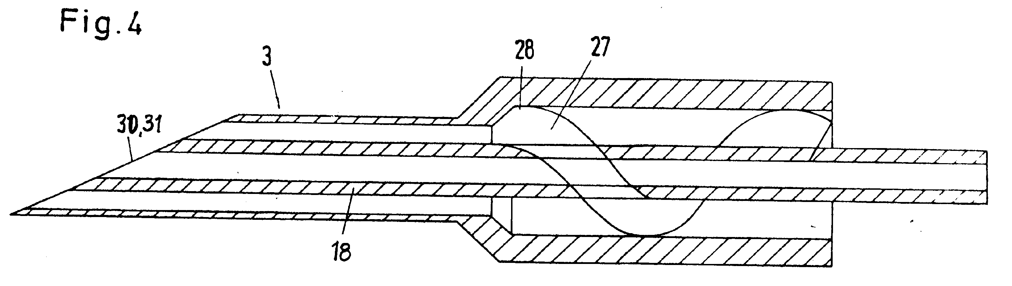

Fig. 4 zeigt eine Ausführungsform der Injektionshohlnadel 3 für eine Injektionsvorrichtung gemäß der vorliegenden Erfindung, bei der der Tubus 18 als Einzelhohlnadel koaxial so gelegt ist, daß seine Mündungsfläche in der Schliffebene 30 der Hohlnadel 3 als der gemeinsamen Ebene 31 mündet. Die innenliegende Einzelhohlnadel 18 ist dabei in Bezug auf die äußere Hohlnadel 3 durch eine wendelartig verlaufende Rippe 27 festgelegt, die in entsprechende Nuten 28 an wenigstens drei Stellen an der Innenwand der Hohlnadel 3 eingreift. Es sind auch andere Befestigungsmöglichkeiten denkbar, wenn nur sichergestellt ist, daß der Tubus 18 seine Lage in Bezug auf die Hohlnadel 3 beibehält.

## Patentansprüche

1. Injektionsvorrichtung zum Injizieren wenigstens zweier miteinander zur Reaktion zu bringender Komponenten in Schläuchen (4,5), mit einer Injektionshohlnadel (3), die durch eine der Zahl der Komponenten entsprechende Anzahl von Kupplungsanschlüssen (6,7) an einem Katheter (1) beaufschlagbar ist,
**dadurch gekennzeichnet,**
**daß** ein Tubus (18,21) als Verlängerung des im Inneren des äußeren Schlauchs (4) befindlichen inneren Schlauchs (5), zumindest im Bereich der Mündung der Injektionshohlnadel (3), koaxial angeordnet ist u. **daß** die zugeführten Komponenten in einer gemeinsamen Ebene (31) münden, in der die Reaktion der Komponenten eingeleitet wird, wobei die gemeinsame Ebene (31) in der Schliffebene (30) der Injektionshohlnadel (3) oder in geringem Abstand davon in der Injektionshohlnadel (3) liegt.

2. Injektionsvorrichtung nach Anspruch 1, dadurch gekennzeichnet, daß die Injektionshohlnadel (3) mit einer am äußeren Schlauch (4) befestigten Hülse (8) verbunden ist, in welcher der innere Schlauch (5) endet.

3. Injektionsvorrichtung nach Anspruch 1 oder 2, dadurch gekennzeichnet, daß die der Zahl der Komponenten entsprechende, im Katheter (1) verlaufende Anzahl von Schläuchen (4,5) über jeweils ein Kupplungsanschluß (6,7) mit einem Griffstück (2) verbunden ist u. das Griffstück (2) in den Katheter (1) verschiebbar ausgebildet ist.

## Claims

1. An injection device for injecting at least two components to be reacted together in flexible tubes (4, 5), comprising a cannula (3) acted upon by a number of coupling connections (6, 7) corresponding to the number of components, on a catheter (1), characterised in that a tube (18, 21) prolonging the inner flexible tube (5) disposed inside the outer flexible tube (4) is disposed coaxially at least in the region of the mouth of the injection cannula (3), and that the supplied components flow out into a common plane (31) in which the reaction between the components is initiated, the common plane (31) being situated in the ground surface (30) of the cannula (3) or at a slight distance therefrom in the cannula (3).

2. An injection device according to claim 1, characterised in that the cannula (3) is connected to a sleeve (8) secured to the outer flexible tube (4), and in which the inner flexible tube (5) ends.

3. An injection device according to claim 1 or 2, characterised in that the number of flexible tubes (4, 5) corresponding to the number of components and extending in the catheter (1) are each connected by a coupling (6, 7) to a handle (2), and the handle (2) is movably disposed in the catheter (1).

## Revendications

1. Dispositif d'injection pour injecter au moins deux composants devant être amenés en réaction, dans des tuyaux (4, 5), comportant une aiguille creuse d'injection (3) qui, par un nombre de raccords de branchement (6, 7) correspondant au nombre de composants, peut être alimentée par un cathéter,
caractérisé
en ce qu'un tube (18, 21), en guise de prolongement du tuyau intérieur (5) se trouvant à l'intérieur du tuyau extérieur (4), est disposé coaxialement au moins dans la zone de l'orifice de l'aiguille creuse d'injection (3), et
en ce que les composants amenés, débouchent dans un plan commun (31) dans lequel est déclenchée la réaction, le plan commun (31) se situant dans le plan d'affûtage (30) de l'aiguille creuse d'injection (3), ou à faible distance de celui-ci, dans l'aiguille creuse d'injection (3).

2. Dispositif d'injection selon la revendication 1, caractérisé en ce que l'aiguille creuse d'injection (3) est reliée à une douille (8) fixée sur le tuyau extérieur (4) et dans laquelle se termine le tuyau intérieur (5).

3. Dispositif d'injection selon la revendication 1 ou 2, caractérisé en ce que le nombre de tuyaux (4, 5) correspondant au nombre de composants, et s'étendant dans le cathéter (1), sont reliés chacun par un raccord de branchement (6, 7), à une pièce de préhension (2), et en ce que la pièce de préhension (2) est conçue de façon à pouvoir s'engager de manière coulissante dans le cathéter (1).
